# EUROPEAN PATENT SPECIFICATION

(11) **EP 3 288 465 B1**
(45) Date of publication and mention of the grant of the patent: **20.02.2019**
(21) Application number: 16720692.9
(22) Date of filing: 28.04.2016
(51) Int. Cl.: A61B 8/00, A61B 8/08, G01C 21/16

(54) **IN-DEVICE FUSION OF OPTICAL AND INERTIAL POSITIONAL TRACKING OF ULTRASOUND PROBES**
VORRICHTUNGSINTERNE FUSION VON OPTISCHER UND INERTER POSITIONSVERFOLGUNG VON ULTRASCHALLSONDEN
FUSION DANS UN DISPOSITIF DU SUIVI OPTIQUE ET INERTIEL DE LA POSITION DE SONDES À ULTRASONS

(30) Priority: 28.04.2015 US 201562153970 P; 28.04.2015 US 201562153974 P; 28.04.2015 US 201562153978 P; 27.04.2016 US 201615140001; 27.04.2016 US 201615140006
(43) Date of publication of application: 07.03.2018
(73) Proprietor: QUALCOMM Incorporated, San Diego, CA 92121-1714 (US)
(72) Inventor: DOS SANTOS MENDONCA, Ricardo Paulo, San Diego, CA 92121-1714 (US); LUNDQVIST, Patrik Nils, San Diego, CA 92121-1714 (US); ATTAR, Rashid Ahmed Akbar, San Diego, CA 92121-1714 (US); JAIN, Rajeev, San Diego, CA 92121-1714 (US); JAGANNATHAN, Padmapriya, San Diego, CA 92121-1714 (US)
(74) Representative: Dunlop, Hugh Christopher
(86) International application number: PCT/US2016/029784
(87) International publication number: WO 2016/176452

(56) References cited:
- WO-A1-2014/134188
- US-A- 6 122 538
- US-A1- 2009 306 509
- US-A1- 2010 198 068
- US-A1- 2012 253 200
- US-A1- 2014 046 186
- US-A1- 2014 243 671
- MUHAMMAD SAADI ET AL: "Performance analysis of optical wireless communication system using pulse width modulation", ELECTRICAL ENGINEERING/ELECTRONICS, COMPUTER, TELECOMMUNICATIONS AND INFORMATION TECHNOLOGY (ECTI-CON), 2013 10TH INTERNATIONAL CONFERENCE ON, IEEE, 15 May 2013 (2013-05-15), pages 1-5, XP032436013, DOI: 10.1109/ECTICON.2013.6559627 ISBN: 978-1-4799-0546-1

## Description

### TECHNICAL FIELD

This disclosure relates to an ultrasonography apparatus, and more particularly to techniques for improving the operability and functionality of the ultrasonography apparatus.

### DESCRIPTION OF THE RELATED TECHNOLOGY

High resolution ultrasonic imaging has been adapted for a large number of medical purposes. Traditionally, the ultrasonic imaging probe is a simple hand-held device that emits and receives acoustic signals. The device is connected by an electrical cable with a console or rack of equipment that provides control signals and power to the probe and that processes acoustic signal data received by the probe and forwarded to the console which processes the received data to produce viewable images of an anatomical feature of interest.

In the present disclosure, techniques are described for improving the operability and functionality of an ultrasonic imaging probe.

US 6122538 A relates to a movable medical imaging device having sensors for measuring the position and orientation of the movable device, which uses recalibration of the gyroscopic sensor to correct drift error. US 2014/0243671 A1 relates to an ultrasound imaging system and method including acquiring motion data from a motion sensing system on a probe while acquiring ultrasound data with the probe, wherein a drift compensation for the probe is calculated using the probe motion data and the probe position data. US 2009/0306509 A1 relates to three-dimensional ultrasonic imaging using conventional two-dimensional ultrasonic imaging apparatus, in which a processor corrects sensor drift and bias by a processor which continuously polls a temperature sensor and corrects the sensor output appropriately.

### SUMMARY

The invention is defined by the independent claims. Further embodiments of the invention are defined by the dependent claims.

The systems, methods and devices of this disclosure each have several innovative aspects, no single one of which is solely responsible for the desirable attributes disclosed herein.

One innovative aspect of the subject matter described in this disclosure relates to an apparatus for ultrasonography that includes one or more ultrasonic transducers, one or more inertial sensors, one or more optical sensors, and a processor communicatively coupled with the one or more ultrasonic transducers, the one or more inertial sensors and the one or more optical sensors. The processor is capable of estimating a position of the apparatus based on a combination of signals received from the one or more ultrasonic transducers, the one or more inertial sensors and the one or more optical sensors.

In some examples, the estimating the position of the apparatus may include processing ultrasound image data from the one or more ultrasonic transducers and determining the position based on the processed ultrasound image data. In some examples, the ultrasound image data may include a series of 2-D image frames and the processed ultrasound image data may include a 3-D image. The processor may be configured to adjust at least one of the 2-D image frames in view of the determined position at a time of obtaining the at least one of the 2-D images.

In some examples, the ultrasound image data may include a series of 2-D image frames and the processed ultrasound image data may include a 3-D image of a first volume. The processor may be configured to determine, with regard to at least one of the 2-D image frames, whether the at least one of the 2-D image frames relates to the first volume or to a different volume.

In some examples, the optical sensor may be optically coupled with one or more optical wireless communication (OWC) emitters of an indoor positioning system. In some examples, the processor may be configured to correct drift error accumulation of the inertial sensors using the combination of signals.

In some examples, the processor may be configured to process image data acquired by one or both of the optical sensors and the ultrasonic transducers so as to select a plurality of landmarks. In some examples, the landmarks may include one or both of: (i) one or more points, edges or corners of ordinary surfaces, fixtures or objects of a room in which the apparatus is to be used to examine a subject; and (ii) one or more anatomical features of the subject, the anatomical features being selected from the group consisting of tissue surfaces, tissue boundaries and image texture of ordinary anatomical or pathological structures of the subject. In some examples, the processor may be configured to calculate the position of the apparatus with respect to the landmarks. In some examples, the processor may be configured to calculate a location of the subject or an anatomical feature of the subject.

In some examples, the processor may be configured to fuse the combination of signals using one or more of visual inertial odometry (VIO) techniques, simultaneous localization and mapping (SLAM) techniques, image registration techniques, or any combination thereof. In some examples, the processor may be configured to process ultrasound image data from the ultrasonic transducer and make a determination of the position of the apparatus from the processed ultrasound image data. In some examples, the processor may be configured to use the determination to provide, to an operator of the apparatus, one or more of: navigational guidance for movement of the imaging probe, notifications based on the determination, identification of anatomical features, identification of pathological structures or any combination thereof.

According to some implementations, a method for ultrasonography includes collecting image data of an environment in which an ultrasonography apparatus is to be operated. The ultrasonography apparatus includes one or more ultrasonic transducers, one or more inertial sensors, one or more optical sensors and a processor communicatively coupled with the one or more ultrasonic transducers, the one or more inertial sensors and the one or more optical sensors, the ultrasonography apparatus being configured to perform noninvasive medical ultrasonography. The method includes estimating, with the processor, a position of the apparatus using a combination of signals received from the one or more ultrasonic transducers, the one or more inertial sensors and the one or more optical sensors.

In some examples, the method includes fusing, with the processor, the combination of signals using one or more of visual inertial odometry (VIO) techniques, simultaneous localization and mapping (SLAM) techniques, image registration techniques, or any combination thereof.

In some examples, the image data may include outputs from one or both of the optical sensors and the ultrasonic transducers, the processor may be configured to process the image data so as to select a plurality of landmarks. The landmarks may include one or both of: (i) one or more points, edges or corners of ordinary surfaces, fixtures or objects of a room in which the apparatus is to be used to examine a subject; and (ii) one or more anatomical features of the subject, the anatomical features being selected from the group consisting of tissue surfaces, tissue boundaries and image texture of ordinary anatomical or pathological structures of the subject. The processor may be configured to determine the position of the ultrasonic transducer with respect to the landmarks.

In some examples, the method may include using the determined position to provide, to an operator of the apparatus, navigational guidance for movement of the imaging probe.

According to some implementations, in a non-transitory computer readable medium having software stored thereon, the software includes instructions for ultrasonography, the instructions causing an apparatus to (i) collect image data of an environment in which an ultrasonography apparatus is to be operated, the ultrasonography apparatus including one or more ultrasonic transducers, one or more inertial sensors, one or more optical sensors and a processor communicatively coupled with the one or more ultrasonic transducers, the one or more inertial sensors and the one or more optical sensors, the ultrasonography apparatus being configured to perform noninvasive medical ultrasonography; and (ii) estimate, with the processor, a spatial position of the apparatus using a combination of signals received from the one or more ultrasonic transducers, the one or more inertial sensors and the one or more optical sensors.

### BRIEF DESCRIPTION OF THE DRAWINGS

Details of one or more implementations of the subject matter described in this specification are set forth in this disclosure and the accompanying drawings. Other features, aspects, and advantages will become apparent from a review of the disclosure. Note that the relative dimensions of the drawings and other diagrams of this disclosure may not be drawn to scale. The sizes, thicknesses, arrangements, materials, etc., shown and described in this disclosure are made only by way of example and should not be construed as limiting. Like reference numbers and designations in the various drawings indicate like elements.
Figure 1 illustrates a hand-held ultrasonic imaging probe, according to an implementation.
Figure 2 illustrates an example of an environment in which the hand-held ultrasonic imaging probe may be operated according to an implementation.
Figure 3 illustrates an example of a method for estimating a position of an ultrasonography apparatus, according to an implementation.
Figure 4 illustrates an example of a method for calibrating an inertial sensor of a ultrasonic imaging probe, according to another implementation.
Figure 5 illustrates an example of a data flow diagram according to an implementation.
Figure 6 illustrates an example of an environment in which the hand-held ultrasonic imaging probe may be operated according to another implementation.

### DETAILED DESCRIPTION

Details of one or more implementations of the subject matter described in this specification are set forth in this disclosure, which includes the description and claims in this document, and the accompanying drawings. Other features, aspects and advantages will become apparent from a review of the disclosure. Note that the relative dimensions of the drawings and other diagrams of this disclosure may not be drawn to scale. The sizes, thicknesses, arrangements, materials, etc., shown and described in this disclosure are made only by way of example and should not be construed as limiting.

The present inventors have developed techniques for improving the portability, operability and functionality of ultrasonic scanners such that they may be used in a greater diversity of physical settings and by a user (care provider) who is not necessarily a specialized ultrasound technician (sonographer). For example, in a related provisional patent application entitled "AUTO-CONFIGURATION OF A DEVICE FOR ULTRASOUND IMAGING", U.S. Provisional Patent Application No 62/153,978, filed on April 28, 2015, owned by the assignee of the present application, techniques are described for largely automating a process of setting up and/or optimizing settings of the ultrasonic probe. As a further example, in a related provisional patent application entitled "IN-DEVICE FUSION OF OPTICAL AND INERTIAL POSITIONAL TRACKING OF ULTRASOUND PROBES", U.S. Provisional Patent Application No 62/153,970, filed on April 28, 2015, owned by the assignee of the present application, techniques are described that enable a hand-held ultrasonic imaging probe to determine its own spatial position using optical and inertial sensors whether or not the probe is being used in a dedicated ultrasound examination room.

The systems, methods and devices of the disclosure each have several innovative aspects, no single one of which is solely responsible for the desirable attributes disclosed herein. One innovative aspect of the subject matter described in this disclosure can be implemented in a portable ultrasonic imaging probe for medical ultrasonography. In some implementations, the portable ultrasonic imaging probe may be hand-held. In some implementations, the portable ultrasonic imaging may be included in or attached to an apparatus such as a robot, or may be or include a wearable device. For example, a sleeve, wearable by a human or robotic operator and/or by a patient or other subject of examination (hereinafter, "subject") may contain one or more ultrasonic transducers, one or more inertial sensors, and/or one or more optical sensors.

In another example, the wearable device may contain one or more ultrasonic transducers communicatively coupled to a processor by way of a wired or wireless interface. Whether or not the wearable sleeve also includes optical sensors, the processor may also be communicatively coupled to one or more inertial sensors of the wearable device and/or one or more optical sensors disposed within an examination room where the wearable device is located. The optical sensors may be configured to capture image data of the wearable device and provide it to the processor, which can use the image data to determine a location of the wearable device. The ultrasonic transducers of the wearable device may capture ultrasound data and send it to the processor, which uses the data to generate an ultrasound volume and also determine a precise location of the wearable device relative to the subject's body.

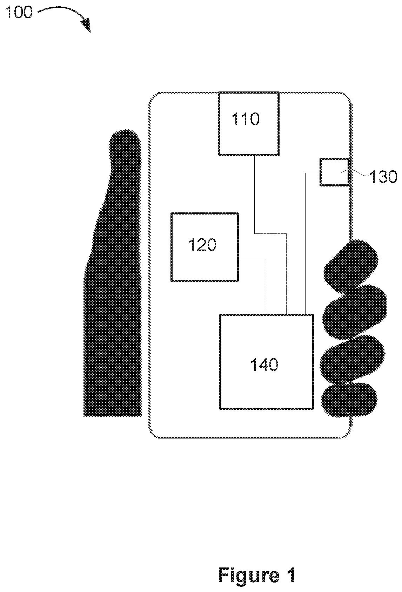

Figure 1 illustrates a hand-held ultrasonic imaging probe, according to an implementation. The apparatus 100 includes an ultrasonic transducer 110, an inertial sensor 120, an optical sensor 130 and a processor 140 communicatively coupled with the ultrasonic transducer 110, the inertial sensor 120, and the optical sensor 130. The processor 140 may be configured to calibrate the inertial sensor 120 using outputs from the optical sensor 130. For example, the processor 140 may be configured to correct for accumulated drift errors of the inertial sensor 120. In some implementations, the hand-held ultrasonic imaging probe may be configured to make a real-time determination of its spatial position with respect to an arbitrary coordinate system using a combination of optical and inertial sensors. As used herein, and in the claims, the terms "spatial position" and "position" refers to a spatial location (e.g., in terms of X, Y and Z coordinate location) in combination with an angular orientation (e.g. roll, pitch and yaw angle) and may be referred to as a 6 degree of freedom (6-DoF) spatial position. As used herein, and in the claims, the term "optical sensor" refers to a device configured to optically detect visible, infrared and or ultraviolet light and/or images thereof, and includes any kind of camera or photodetector.

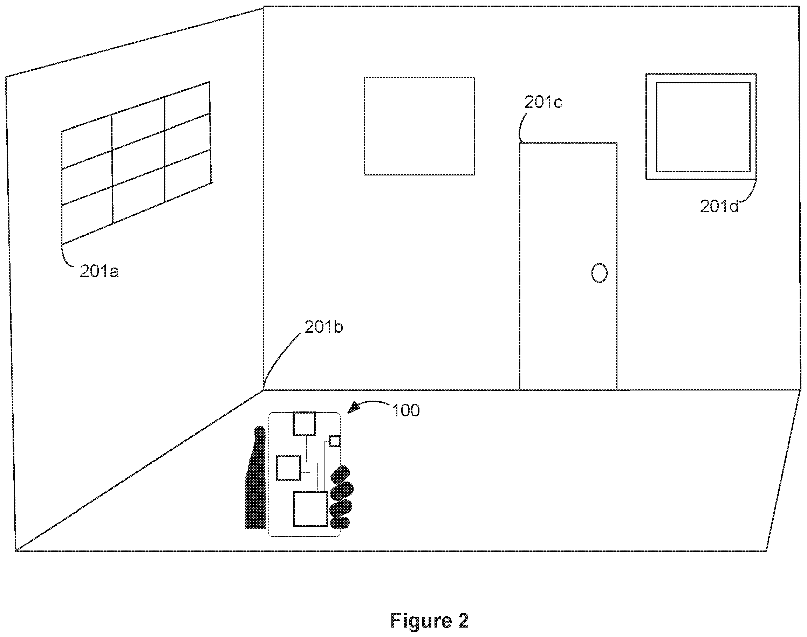

Figure 2 illustrates an example of an environment in which the hand-held ultrasonic imaging probe may be operated according to an implementation. Where the apparatus 100 includes the processor 140 communicatively coupled with one or more optical sensors 130, the processor 140 may be configured to collect image data of an environment (for example, an examining room) in which a subject is to be examined using the apparatus. The processor 140 may be configured to process the acquired environmental image data so as to select a plurality of fixed "landmarks" in the vicinity of the probe. These landmarks may include visually well-defined points, edges or corners of surfaces, fixtures, and/or objects of an ordinary room in which an operator wishes to perform an ultrasonic exam such as corners 201a, 201b, 201c and 201d. The processor may be configured to calculate, in real time, the probe's X, Y and Z location as well as the probe's pitch, yaw, and roll orientation with respect to these landmarks. Moreover, the processor may be configured to calculate, in real time, location of the subject or an anatomical feature of the subject.

As indicated above, the processor 140 may also be communicatively coupled with at least one inertial sensor 120. The inertial sensor 120 may be configured to measure translational and rotational motion of the apparatus 100. The inertial sensor 120 may be configured as or include an accelerometer, a gyroscope, a MEMS inertial sensor, etc. Using visual inertial odometry (VIO) techniques, such as those which have been developed in the field of robotics, the processor may be configured to estimate, in real-time, the probe's spatial position notwithstanding that some or all of the landmarks 201 may be, from time to time, obscured from view of the optical sensors, and notwithstanding normal inertial sensor drift error accumulation. Alternatively, or in addition, simultaneous localization and mapping (SLAM) techniques and image registration techniques may be used. As a result, the combination of optical sensor data and inertial sensor data will enable a reasonably accurate estimation of the probe's spatial position. Thus, the estimation of the probe's position may be based on a combination of data from the inertial sensors and the optical sensors. Alternatively, or in addition, the estimation may be based on a prior position fix determined via optical sensors updated with current data from the inertial sensors.

In an implementation, the processor 140 may be configured to receive data inputs from the inertial sensor 120 and the optical sensor 130 and/or the ultrasonic transducer, and to use the received data inputs to determine the spatial position of the apparatus 100. For example, the processor may be configured to estimate a 6-DoF spatial position of the apparatus using a combination of outputs from two or more of the ultrasonic transducer 110, the inertial sensor 120 and the optical sensor 130. Moreover, the processor may be configured to correct drift error accumulation of the inertial sensor 120 using the combination of outputs. The processor 140 may be further configured to process ultrasound image data from the ultrasonic transducer 110, using the determined spatial position of the apparatus 100. For example, a series of sequential 2-D image frames (obtained for example at a rate of 30 frames per second or higher) may be collated to form a 3-D image, after appropriate adjustment of each 2-D image in view of the respective spatial position of the apparatus 100 at the time of obtaining each respective 2-D image.

In an implementation, the processor may be configured to process image data acquired by one or both of the optical sensor and the ultrasonic transducer so as to select a plurality of landmarks. As indicated above, in some implementations, the landmarks may include points, edges or corners of ordinary surfaces, fixtures, and/or objects of a room in which in which the apparatus is to be used to examine a subject. In addition, or alternatively, the landmarks may include one or more anatomical features of the subject, the anatomical features including one or more of tissue surfaces, tissue boundaries or image texture of ordinary anatomical or pathological structures of the subject.

In an implementation, the apparatus may also include one or more optical sensors that are directed towards the subject. Signals from the optical sensors may better allow the apparatus to track its position relative to the subject's body.

In another implementation, the apparatus may include one or more optical sensors directed towards the environment the apparatus is located in, and one or more optical sensors directed towards the subject. This may better allow the apparatus to determine a position of the apparatus relative to the environment and also determine the position of the apparatus to the body. As a result, even if the subject moves, the ultrasound volume generation may be substantially unimpaired because the apparatus is aware of its location with respect to the environment as well as with respect to the subject. Otherwise, if the subject moved and the apparatus only had its position relative to the environment, then the apparatus might inadvertently add ultrasound data to an incorrect ultrasound volume.

As a result, outputs of an ultrasonic scan performed by the probe may be processed, in light of the determined spatial position of the probe, to determine the relative position, in three-dimensional space, of each of a sequence of 2-D images.

In an implementation, the processor 140 may be configured to use the determined spatial position to provide, to an operator of the apparatus, navigational guidance for movement of the hand-held ultrasonic imaging probe.

Knowledge of the relative position of each 2-D image with respect to an arbitrary reference frame may enable one or more of the following applications, for example: (i) the creation of more accurate three dimensional ultrasound volumes from two-dimensional ultrasound images; (ii) the overlaying of each image onto an optical or alternative image of the subject, with accurate anatomical registration of internal structures; (iii) the combination of multiple two-dimensional images into another two-dimensional image with better quality and larger anatomical coverage, and (iv) the provision to the ultrasound operator of navigational guidance for probe movement.

Integration of the processor, the optical sensor, and the inertial sensor component as part of the hand-held ultrasonic imaging probe, enables a positional tracking function for the probe that is cost efficient and compact. The proposed techniques do not require external equipment such as magnetic trackers nor special room preparation as needed by tracking systems that rely on depth images or external vision sensors. Neither do the techniques require the application of cumbersome or conspicuous visual markers on the probe and/or the subject.

In contrast to the present disclosure, known optical-only systems demand that a large number-often hundreds-of visually conspicuous features (such as points, corners, colored patches, markers) are visible in the environment and that such features can be reliably matched between subsequent frames. Inertial sensors, on the other hand, are operable in the absence of any external visual reference, but they quickly lose absolute accuracy as the tracked device moves.

In accordance with the present disclosure, inertial sensors provide good relative positional accuracy over short periods of time during which landmarks may be obscured from the field of view of the optical sensors. This knowledge is used to accurately estimate, substantially continuously, the spatial position of the camera during an ultrasound scan. As a result, a need for a large number of specially configured conspicuous visual features in the environment of the ultrasound scan can be eliminated. Consequently, the ultrasonic imaging probe may be used to obtain real time 3-D images even in environments that have not been equipped for ultrasound imaging. For example, the present disclosure contemplates the ultrasonic imaging probe may be used in an ordinary room in which a subject may be examined such as a doctor's office, emergency room, or in a subject's home.

The application of integrated optical and inertial positional tracking is particularly apt for establishing the spatial position and orientation of ultrasound probes, because in such applications there is a reasonable expectation that the probe will be held in a particular manner by the operator, so that the optical sensors can be strategically placed on the device to ensure maximum visibility of the external environment.

The presently disclosed techniques bring many benefits to the medical diagnosis and to the user experience of the ultrasound operator and subject. In some implementations, for example, the techniques enable production of accurate three-dimensional models of a subject's anatomy and pathological structures without the use of external devices and room preparation. As a result, field application of ultrasonic imaging, outside a clinical setting may be enabled. Such 3-D models may be used in real time for a more accurate subject diagnosis or assessment, and also may be stored for future comparison against new two or three dimensional data.

As a further example, in some implementations obtained ultrasound images may be overlaid against an optical image of the subject with the appropriate anatomical alignment. Such overlay may be displayed on a separate screen or transmitted wirelessly or otherwise to a headmounted display (HMD) which would overlay the ultrasound image against a live image of the subject. In an implementation, a position of the HMD relative to the probe may be obtained and images displayed by the HMD may be adjusted based on the HMD's position relative to the probe's position. For example, the HMD may include optical and/or inertial sensors from which its 6-DoF spatial position may be obtained. Based on the obtained 6-DoF spatial position, images displayed by the HMD may be changed accordingly. For example, as an operator wearing the HMD moves around a subject's body, displayed images of the ultrasonic volume may be observed from multiple angles. In some implementations, the probe device may be a wearable sleeve with multiple ultrasonic transducers, optical and/or inertial sensors, communicatively coupled with the HMD, enabling an operator wearing the HMD to obtain a rich, three dimensional, view of a subject's anatomy or pathological structure. The multiple ultrasonic transducers, optical and/or inertial sensors may be calibrated to determine, for example, their proximity to one another prior to and/or during examination of the subj ect.

As a yet further example, in some implementations navigational guidance for moving the probe may be provided, with an objective of aiding the ultrasound operator in the task of placing the probe for optimal image acquisition. This may enable the use of ultrasound imaging by operators with less experience and training, thereby facilitating the adaption of ultrasound imaging technology.

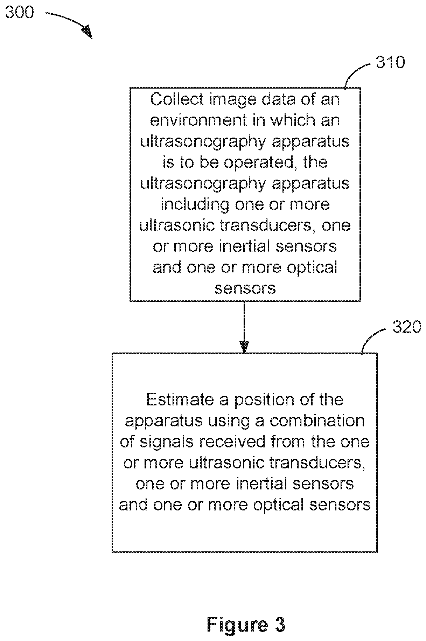

In some implementations, the integration of optical with inertial measurements may include use of an extended Kalman filter (EKF) which would optimally combine measurements from each type of sensor into an overall coherent estimation of the probes position and orientation. Figure 3 illustrates an example of a method for estimating a position of an ultrasonography apparatus. As described hereinabove, the ultrasonography apparatus may include one or more ultrasonic transducers, one or more inertial sensors, one or more optical sensors and a processor communicatively coupled with the one or more ultrasonic transducers, the one or more inertial sensors and the one or more optical sensors. In the illustrated implementation, method 300 includes a block 310 for collecting, with the optical sensor and/or the ultrasonic transducer, image data of an environment in which the ultrasonic imaging probe is to be operated.

The method proceeds, at block 320, with estimating, using the processor, a position of the apparatus using a combination of signals received from the one or more of the ultrasonic transducers, the one or more inertial sensors and the one or more optical sensors. For example, the processor may use outputs from the optical sensor and/or the ultrasonic transducer to correct for accumulated drift errors of the inertial sensor.

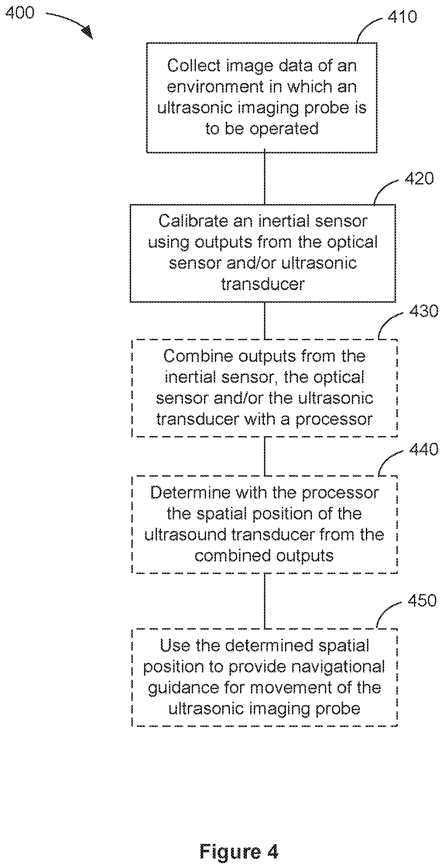

Figure 4 illustrates an example of a method for calibrating an inertial sensor of a hand-held ultrasonic imaging probe and, according to an implementation. As described hereinabove, the imaging probe may include an ultrasonic transducer, an inertial sensor and a processor communicatively coupled with the ultrasonic transducer the inertial sensor and the optical sensor. In the illustrated implementation, method 400 includes a block 410 for collecting, with one or both of the optical sensor and the ultrasonic transducer, image data of an environment in which the ultrasonic imaging probe is to be operated.

The method proceeds, at block 420, with calibrating, using the processor, the inertial sensor, using outputs from the optical sensor and/or ultrasonic transducer. For example, the processor may use the outputs to correct for accumulated drift errors of the inertial sensor.

Optionally, in some implementations the method 400 may proceed at block 430 with combining, with the processor, outputs from the inertial sensor and from one or both of the optical sensor and the ultrasonic transducer. As a further optional step, the method 400 may proceed at block 440 with determining with the processor the spatial position of the ultrasound transducer with the combined outputs obtained at block 430. In a yet further optional step, the method 400 may proceed at block 450 with using the spatial position, determined at block 440, to provide navigational guidance for movement of the ultrasonic imaging probe. Navigational guidance may be provided to an operator using the ultrasonic imaging probe to perform noninvasive medical ultrasonography.

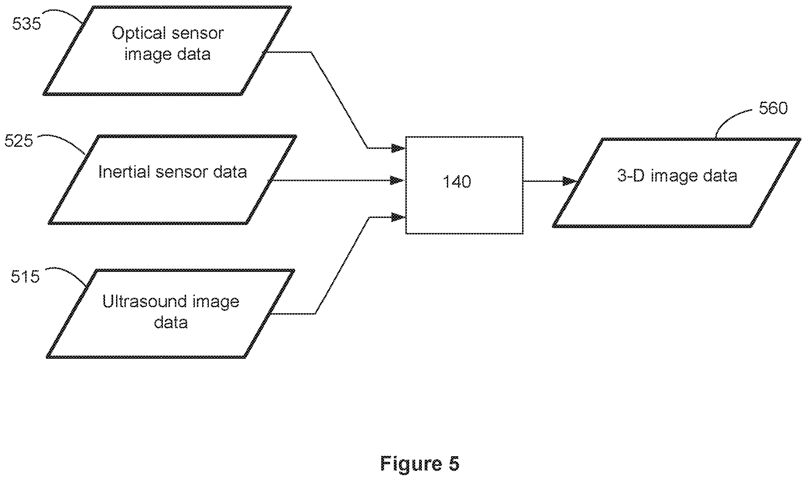

In an implementation, the processor may be configured to process ultrasound image data from the ultrasonic transducer and calibrate the estimated 6-DoF spatial position of the apparatus 100 using the processed ultrasound image data optical sensor image data. Figure 5 illustrates an example of a data flow diagram according to an implementation. In the illustrated implementation the processor 140 processes ultrasound image data 515, inertial sensor data 525, and optical sensor image data 535. As a result, outputs from each of the ultrasonic transducer 110, the inertial sensor 120, and the optical sensor 130 may be fused so as to obtain a more accurately calibrated estimation of the spatial position of the apparatus 100.

Where the ultrasound image data 515 includes a series of 2-D image frames and the processed ultrasound image data includes a 3-D image, the processor may be configured to adjust one or more of the 2-D image frames in view of the estimated 6-DoF spatial position at the time of obtaining each respective 2-D image. For example where the estimated 6-DoF spatial position at a time corresponding to a 2-D image frame (i) is different from the estimated 6-DoF spatial position at a time corresponding to a 2-D image frame (i+1), one or both of the respective 2-D image frames may be adjusted to compensate for the difference. As a result, temporal series of 2-D images may be more accurately combined to compute 3-D image data 560.

In an implementation, the processor may be configured to make a determination whether or not an obtained 2-D image frame relates to a first volume under examination or a different volume. For example, where an operator interrupts and then resumes use of the apparatus (e.g., by lifting if up from a first location and then setting it down at a second location), the operator may or may not intend that the first location and the second location be substantially identical. Upon resumption of use of the apparatus, the processor may be configured to determine, with regard to a newly received 2-D image frame, whether data from the 2-D image frame should be merged with previously received image frame data (because the first location and the second location are substantially identical) or not merged (because the first location and the second location are not substantially identical). For example, the processor may be configured to determine a difference between two or more 2-D image frames and compare the difference to a threshold to determine if the images relate to approximately the same location. As a further example, the processor may be configured to compare the 6-DoF spatial position, as well as operator settings of the ultrasound probe (e.g., frequency and gain, image depth and signal processing filter parameters) associated with the two or more 2-D image frames to determine if they should be associated with the same volume.

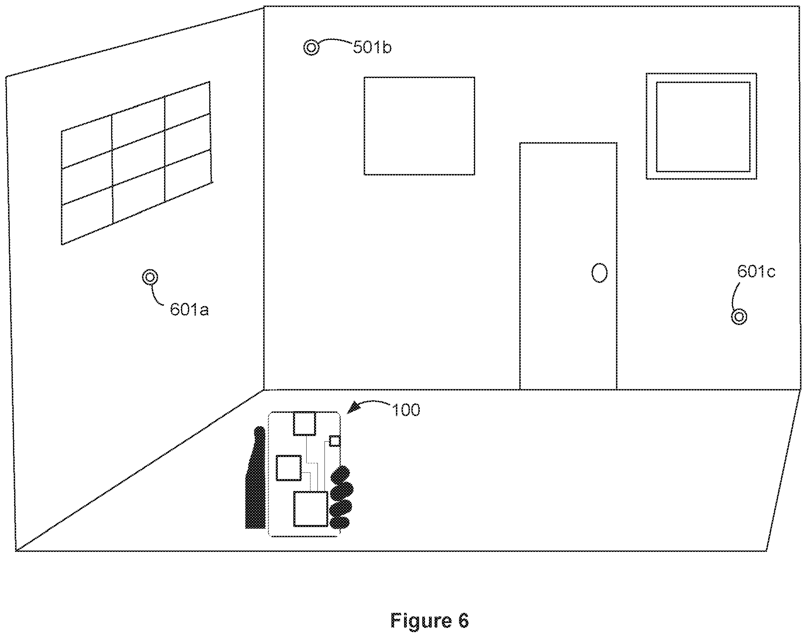

Figure 6 illustrates an example of an environment in which the hand-held ultrasonic imaging probe may be operated according to another implementation. Where the apparatus 100 includes the processor 140 communicatively coupled with one or more optical sensors 130, the processor 140 may be configured to collect image data of an environment (for example, an examining room) in which a subject is to be examined using the apparatus. In the illustrated implementation, the examining room includes a plurality of optical emitters 501 configured for optical wireless communication (OWC). The optical sensors 130 may be optically coupled so as to receive signals from the emitters 601, which may be configured as part of an indoor positioning system (IPS). In an implementation, the optical emitters are configured for visible light communication (VLC). In other implementations, the optical emitters may be configured for communication in the infrared and/or ultraviolet light wavelengths. The IPS may enable the processor to calculate, in real time, the probe's X, Y and Z location as well as the probe's pitch, yaw, and roll orientation with respect to the optical emitters 601. Moreover, the processor may be configured to calculate, in real time, location of the subject or an anatomical feature of the subject, with or without use of an inertial sensor.

As indicated above, the processor 140 may also be communicatively coupled with at least one inertial sensor 120. The inertial sensor 120 may be configured to measure translational and rotational motion of the apparatus 100. Using VIO techniques, the processor may be configured to estimate, in real-time, the probe's spatial position notwithstanding that some or all of the optical emitters 601 may be obscured from view of the optical sensors, and notwithstanding normal inertial sensor drift error accumulation.

Thus, a smart device for ultrasound imaging has been disclosed that is configured as an ultrasonic imaging probe that includes an inertial sensor and an optical sensor where the processor is configured to calibrate the inertial sensor using outputs from the optical sensor. It will be appreciated that a number of alternative configurations and fabrication techniques may be contemplated.

As used herein, a phrase referring to "at least one of' a list of items refers to any combination of those items, including single members. As an example, "at least one of: a, b, or c" is intended to cover: a, b, c, a-b, a-c, b-c, and a-b-c.

The various illustrative logics, logical blocks, modules, circuits and algorithm processes described in connection with the implementations disclosed herein may be implemented as electronic hardware, computer software, or combinations of both. The interchangeability of hardware and software has been described generally, in terms of functionality, and illustrated in the various illustrative components, blocks, modules, circuits and processes described above. Whether such functionality is implemented in hardware or software depends upon the particular application and design constraints imposed on the overall system.

The hardware and data processing apparatus used to implement the various illustrative logics, logical blocks, modules and circuits described in connection with the aspects disclosed herein may be implemented or performed with a general purpose single- or multi-chip processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general purpose processor may be a microprocessor or any conventional processor, controller, microcontroller, or state machine. A processor also may be implemented as a combination of computing devices, e.g., a combination of a DSP and a microprocessor, a plurality of microprocessors, one or more microprocessors in conjunction with a DSP core, or any other such configuration. In some implementations, particular processes and methods may be performed by circuitry that is specific to a given function.

In one or more aspects, the functions described may be implemented in hardware, digital electronic circuitry, computer software, firmware, including the structures disclosed in this specification and their structural equivalents thereof, or in any combination thereof. Implementations of the subject matter described in this specification also can be implemented as one or more computer programs, i.e., one or more modules of computer program instructions, encoded on a computer storage media for execution by or to control the operation of data processing apparatus.

If implemented in software, the functions may be stored on or transmitted over as one or more instructions or code on a computer-readable medium, such as a non-transitory medium. The processes of a method or algorithm disclosed herein may be implemented in a processor-executable software module which may reside on a computer-readable medium. Computer-readable media include both computer storage media and communication media including any medium that can be enabled to transfer a computer program from one place to another. Storage media may be any available media that may be accessed by a computer. By way of example, and not limitation, non-transitory media may include RAM, ROM, EEPROM, CD-ROM or other optical disk storage, magnetic disk storage or other magnetic storage devices, or any other medium that may be used to store desired program code in the form of instructions or data structures and that may be accessed by a computer. Also, any connection can be properly termed a computer-readable medium. Disk and disc, as used herein, includes compact disc (CD), laser disc, optical disc, digital versatile disc (DVD), floppy disk, and blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and instructions on a machine readable medium and computer-readable medium, which may be incorporated into a computer program product.

Various modifications to the implementations described in this disclosure may be readily apparent to those skilled in the art, and the generic principles defined herein may be applied to other implementations without departing from the spirit or scope of this disclosure. Thus, the claims are not intended to be limited to the implementations shown herein, but are to be accorded the widest scope consistent with this disclosure, the principles and the novel features disclosed herein. Additionally, as a person having ordinary skill in the art will readily appreciate, the terms "upper" and "lower", "top" and bottom", "front" and "back", and "over", "on", "under" and "underlying" are sometimes used for ease of describing the figures and indicate relative positions corresponding to the orientation of the figure on a properly oriented page, and may not reflect the proper orientation of the device as implemented.

Certain features that are described in this specification in the context of separate implementations also can be implemented in combination in a single implementation. Conversely, various features that are described in the context of a single implementation also can be implemented in multiple implementations separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed to achieve desirable results. Further, the drawings may schematically depict one more example processes in the form of a flow diagram. However, other operations that are not depicted can be incorporated in the example processes that are schematically illustrated. For example, one or more additional operations can be performed before, after, simultaneously, or between any of the illustrated operations. In certain circumstances, multitasking and parallel processing may be advantageous. Moreover, the separation of various system components in the implementations described above should not be understood as requiring such separation in all implementations, and it should be understood that the described program components and systems can generally be integrated together in a single software product or packaged into multiple software products. Additionally, other implementations are within the scope of the following claims. In some cases, the actions recited in the claims can be performed in a different order and still achieve desirable results.

## Claims

1. An apparatus for ultrasonography, the apparatus comprising:
an imaging probe including one or more ultrasonic transducers (110), one or more inertial sensors (120) and one or more optical sensors (130); and
a processor (140) communicatively coupled with the one or more ultrasonic transducers, the one or more inertial sensors and the one or more optical sensors;
**characterised in that** the processor is capable of:
calibrating outputs of the one or more inertial sensors by correcting for drift error accumulation of the one or more inertial sensors using a combination of signals received from the one or more ultrasonic transducers and the one or more optical sensors and
estimating a position of the imaging probe based on the calibrated outputs of the one or more inertial sensors.

2. The apparatus of claim 1, wherein the estimating the position of the imaging probe comprises:
processing ultrasound image data from the one or more ultrasonic transducers; and
determining the position based on the processed ultrasound image data.

3. The apparatus of claim 2, wherein:
the ultrasound image data includes a series of 2-D image frames and the processed ultrasound image data includes a 3-D image, and
the processor is configured to adjust at least one of the 2-D image frames in view of the determined position at a time of obtaining the at least one of the 2-D images.

4. The apparatus of claim 2, wherein:
the ultrasound image data includes a series of 2-D image frames and the processed ultrasound image data includes a 3-D image of a first volume, and
the processor is configured to determine, with regard to at least one of the 2-D image frames, whether the at least one of the 2-D image frames relates to the first volume or to a different volume.

5. The apparatus of claim 1, wherein the optical sensor is optically coupled with one or more optical wireless communication emitters of an indoor positioning system.

6. The apparatus of claim 1, wherein the processor is configured to process image data acquired by one or both of the optical sensors and the ultrasonic transducers so as to select a plurality of landmarks.

7. The apparatus of claim6, wherein the landmarks include one or both of:
one or more points, edges or corners of ordinary surfaces, fixtures or objects of a room in which the apparatus is to be used to examine a subject; and
one or more anatomical features of the subject, the anatomical features being selected from the group consisting of tissue surfaces, tissue boundaries and image texture of ordinary anatomical or pathological structures of the subject.

8. The apparatus of claim 7, wherein the processor is configured to calculate the position of the imaging probe with respect to the landmarks, a location of the subject or an anatomical feature of the subject..

9. The apparatus of claim 1, wherein the processor is configured to fuse the combination of signals using one or more of visual inertial odometry techniques, simultaneous localization and mapping techniques, image registration techniques, or any combination thereof.

10. The apparatus of claim 9, wherein the processor is configured to:
process ultrasound image data from the ultrasonic transducer; and
make a determination of the position of the imaging probe from the processed ultrasound image data.

11. A method for ultrasonography, the method comprising:
collecting (310, 410) image data of an environment in which an ultrasonography apparatus is to be operated, the ultrasonography apparatus including a processor and an imaging probe that includes one or more ultrasonic transducers, one or more inertial sensors and one or more optical sensors, the processor being communicatively coupled with the one or more ultrasonic transducers, the one or more inertial sensors and the one or more optical sensors, the ultrasonography apparatus being configured to perform noninvasive medical ultrasonography; **characterised in that** the method further comprises:
calibrating (420) outputs of the one or more inertial sensors by correcting for drift error accumulation of the one or more inertial sensors using a combination of signals received from the one or more ultrasonic transducers and the one or more optical sensors; and
estimating, with the processor, a position of the imaging probe based on the calibrated outputs of the one or more inertial sensors.

12. The method of claim 11, further comprising:
fusing, with the processor, the combination of signals using one or more of visual inertial odometry techniques, simultaneous localization and mapping techniques, image registration techniques, or any combination thereof.

13. The method of claim 11, wherein:
the image data includes outputs from one or both of the optical sensors and the ultrasonic transducers;
the processor is configured to process the image data so as to select a plurality of landmarks, the landmarks including one or both of:
one or more points, edges or corners of ordinary surfaces, fixtures or objects of a room in which the apparatus is to be used to examine a subject; and
one or more anatomical features of the subject, the anatomical features being selected from the group consisting of tissue surfaces, tissue boundaries and image texture of ordinary anatomical or pathological structures of the subject; and
the processor is configured to determine the position of the imaging probe with respect to the landmarks.

14. The method of claim 11, further comprising using (450) the determined position to provide, to an operator of the apparatus, navigational guidance for movement of the imaging probe.

15. A non-transitory computer readable medium having software stored thereon, the software including instructions for ultrasonography, the instructions causing an apparatus to perform a method in accordance with any of claims 11 to 14.

## Patentansprüche

1. Vorrichtung für Ultraschalluntersuchungen, wobei die Vorrichtung umfasst:
eine Bilderfassungssonde mit einem oder mehreren Ultraschallwandlern (110);
einen oder mehrere Trägheitssensoren (120) und einen oder mehrere optische Sensoren (130); und
einen Prozessor (140), der kommunikativ mit dem einen oder den mehreren Ultraschallwandlern, mit dem einen oder den mehreren Trägheitssensoren und mit dem einen oder den mehreren optischen Sensoren gekoppelt ist;
**dadurch gekennzeichnet, dass**
der Prozessor in der Lage ist zum:
Kalibrieren von Ausgaben des einen oder der mehreren Trägheitssensoren mittels Korrigieren von Driftfehler-Akkumulation des einen oder der mehreren Trägheitssensoren unter Verwendung einer Kombination von Signalen, die von dem einen oder den mehreren Ultraschallwandlern und von dem einen oder den mehreren optischen Sensoren empfangen wurden; und
Schätzen einer Position der Bilderfassungssonde basierend auf den kalibrierten Ausgaben des einen oder der mehreren Trägheitssensoren.

2. Die Vorrichtung nach Anspruch 1, wobei das Schätzen der Position der Bilderfassungssonde Folgendes umfasst:
Verarbeiten von Ultraschallbilddaten von dem einen oder den mehreren Ultraschallwandlern; und
Bestimmen der Position basierend auf den verarbeiteten Ultraschallbilddaten.

3. Die Vorrichtung nach Anspruch 2, wobei die Ultraschallbilddaten eine Reihe von 2-D-Bildframes umfassen und wobei die verarbeiteten Ultraschallbilddaten ein 3-D-Bild umfassen; und
wobei der Prozessor eingerichtet ist, mindestens einen der 2-D-Bildframes in Anbetracht der ermittelten Position zu einem Zeitpunkt des Erfassens des mindestens einen der 2-D-Bilder anzupassen.

4. Die Vorrichtung nach Anspruch 2, wobei die Ultraschallbilddaten eine Reihe von 2-D-Bildframes umfassen und wobei die verarbeiteten Ultraschallbilddaten ein 3-D-Bild eines ersten Volumens umfassen; und
wobei der Prozessor eingerichtet ist, bezüglich mindestens eines der 2-D-Bildframes zu bestimmen, ob sich der mindestens eine der 2-D-Bildframes auf das erste Volumen oder auf ein anderes Volumen bezieht.

5. Die Vorrichtung nach Anspruch 1, wobei der optische Sensor optisch mit einem oder mehreren optischen, drahtlosen Kommunikationsemittern eines Indoor-Positionierungssystems gekoppelt ist.

6. Die Vorrichtung nach Anspruch 1, wobei der Prozessor eingerichtet ist, Bilddaten zu verarbeiten, die von dem einen oder von den beiden optischen Sensoren und von den Ultraschallwandlern erfasst wurden, um eine Vielzahl von Orientierungspunkten auszuwählen.

7. Die Vorrichtung nach Anspruch 6, wobei die Orientierungspunkte mindestens einen der beiden folgenden Aspekte umfassen:
einen oder mehrere Punkte, Kanten oder Ecken von gewöhnlichen Oberflächen, Einbauten oder Objekten eines Raumes, in dem die Vorrichtung verwendet werden soll, um einen Gegenstand zu untersuchen; und
ein oder mehrere anatomische Merkmale des Gegenstandes, wobei die anatomischen Merkmale aus der Gruppe bestehend aus Gewebeoberflächen, Gewebegrenzen und Bildtextur von gewöhnlichen anatomischen oder pathologischen Strukturen des Gegenstandes ausgewählt werden.

8. Die Vorrichtung nach Anspruch 7, wobei der Prozessor eingerichtet ist zum Berechnen der Position der Bilderfassungssonde in Bezug auf die Orientierungspunkte, auf eine Position des Gegenstandes oder auf ein anatomisches Merkmal des Gegenstandes.

9. Die Vorrichtung nach Anspruch 1, wobei der Prozessor eingerichtet ist, die Kombination von Signalen zu koppeln, und zwar unter Verwendung einer oder mehrerer visueller Trägheits-Odometrietechniken, gleichzeitiger Lokalisierungs- und Abbildungstechniken, Bildregistrierungstechniken oder jedweder Kombination hieraus.

10. Die Vorrichtung nach Anspruch 9, wobei der Prozessor eingerichtet ist zum: Verarbeiten von Ultraschallbilddaten von dem Ultraschallwandler; und
Bestimmen der Position der Bilderfassungssonde aus den bearbeiteten Ultraschallbilddaten.

11. Verfahren für Ultraschalluntersuchungen, wobei das Verfahren Folgendes aufweist:
Sammeln (310, 410) von Bilddaten einer Umgebung, in der eine Vorrichtung für Ultraschalluntersuchungen verwendet werden soll, wobei die Ultraschallvorrichtung Folgendes aufweist:
einen Prozessor und eine Bilderfassungssonde mit einem oder mehreren Ultraschallwandlern, einen oder mehrere Trägheitssensoren und einen oder mehrere optische Sensoren,
wobei der Prozessor kommunikativ mit dem einen oder den mehreren Ultraschallwandlern, mit dem einen oder den mehreren Trägheitssensoren und mit dem einen oder den mehreren optischen Sensoren gekoppelt ist, wobei die Ultraschallvorrichtung eingerichtet ist, nicht-invasive, medizinische Ultraschalluntersuchungen durchzuführen;
**dadurch gekennzeichnet, dass** das Verfahren weiterhin aufweist:
Kalibrieren (420) von Ausgaben des einen oder der mehreren Trägheitssensoren mittels Korrigieren von Driftfehler-Akkumulation des einen oder der mehreren Trägheitssensoren unter Verwendung einer Kombination von Signalen, die von dem einen oder den mehreren Ultraschallwandlern und von dem einen oder den mehreren optischen Sensoren empfangen wurden; und
Schätzen, mit dem Prozessor, einer Position der Bilderfassungssonde basierend auf den kalibrierten Ausgaben des einen oder der mehreren Trägheitssensoren.

12. Das Verfahren nach Anspruch 11, welches weiterhin aufweist:
Koppeln, mit dem Prozessor, der Kombination von Signalen unter Verwendung eines oder mehrerer visueller Trägheits-Odometrietechniken, simultaner Lokalisierungs- und Abbildungstechniken, Bildregistrierungstechniken oder jedweder Kombination hieraus.

13. Das Verfahren nach Anspruch 11, wobei die Bilddaten Ausgaben von dem einem oder von den beiden der optischen Sensoren und von den Ultraschallwandlern umfassen;
wobei der Prozessor eingerichtet ist, die Bilddaten zu verarbeiten, so dass eine Vielzahl von Orientierungspunkten ausgewählt wird, wobei die Orientierungspunkte mindestens einen der beiden folgenden Aspekte umfassen:
einen oder mehrere Punkte, Kanten oder Ecken von gewöhnlichen Oberflächen, Einbauten oder Objekten eines Raumes, in dem die Vorrichtung verwendet werden soll, um einen Gegenstand zu untersuchen; und
ein oder mehrere anatomische Merkmale des Gegenstandes, wobei die anatomischen Merkmale aus der Gruppe bestehend aus Gewebeoberflächen, Gewebegrenzen und Bildtextur von gewöhnlichen anatomischen oder pathologischen Strukturen des Gegenstandes ausgewählt werden; und
wobei der Prozessor eingerichtet ist, die Position der Bilderfassungssonde bezüglich der Orientierungspunkte zu bestimmen.

14. Das Verfahren nach Anspruch 11, das ferner das Verwenden (450) der bestimmten Position aufweist, um einem Benutzer der Vorrichtung Navigationshilfen für eine Bewegung der Bilderfassungssonde bereitzustellen.

15. Nichtflüchtiges, computerlesbares Medium, auf dem Software gespeichert ist, wobei die Software Anweisungen für Ultraschalluntersuchungen umfasst, wobei die Anweisungen die Vorrichtung zur Durchführung eines Verfahrens nach einem der Ansprüche 11 bis 14 veranlassen.

## Revendications

1. Un appareil d'échographie, l'appareil comprenant :
une sonde d'imagerie comprenant un ou plusieurs transducteurs à ultrasons (110), un ou plusieurs capteurs inertiels (120) et un ou plusieurs capteurs optiques (130), et
un processeur (140) couplé en communication aux un ou plusieurs transducteurs à ultrasons, aux un ou plusieurs capteurs inertiels et aux un ou plusieurs capteurs optiques, **caractérisé en ce que** le processeur est capable de :
calibrer des sorties des un ou plusieurs capteurs inertiels par la correction d'une accumulation d'erreurs de dérive des un ou plusieurs capteurs inertiels au moyen d'une combinaison de signaux reçus des un ou plusieurs transducteurs à ultrasons et des un ou plusieurs capteurs optiques, et
estimer une position de la sonde d'imagerie en fonction des sorties calibrées des un ou plusieurs capteurs inertiels.

2. L'appareil selon la Revendication 1, où l'estimation de la position de la sonde d'imagerie comprend :
le traitement de données d'images par ultrasons provenant des un ou plusieurs transducteurs à ultrasons, et
la détermination de la position en fonction des données d'images par ultrasons traitées.

3. L'appareil selon la Revendication 2, où :
les données d'images par ultrasons comprennent une série de trames d'images en 2D et les données d'images par ultrasons traitées comprennent une image en 3D, et
le processeur est configuré de façon à ajuster au moins une des trames d'images en 2D en fonction de la position déterminée au moment de l'obtention de la au moins une des images en 2D.

4. L'appareil selon la Revendication 2, où :
les données d'images par ultrasons comprennent une série de trames d'images en 2D et les données d'images par ultrasons traitées comprennent une image en 3D d'un premier volume, et
le processeur est configuré de façon à déterminer, en ce qui concerne au moins une des trames d'images en 2D, si la au moins une des trames d'images en 2D porte sur le premier volume ou sur un volume différent.

5. L'appareil selon la Revendication 1, où le capteur optique est couplé optiquement à un ou plusieurs émetteurs de communication sans fil optiques d'un système de positionnement intérieur.

6. L'appareil selon la Revendication 1, où le processeur est configuré de façon à traiter des données d'images acquises par un élément ou les deux parmi les capteurs optiques et les transducteurs à ultrasons de façon à sélectionner une pluralité de points de repère.

7. L'appareil selon la Revendication 6, où les points de repère comprennent un élément ou les deux parmi :
un ou plusieurs points, bords ou angles de surfaces ordinaires, accessoires ou objets d'une pièce dans laquelle l'appareil est destiné à être utilisé de façon à examiner un sujet, et
une ou plusieurs caractéristiques anatomiques du sujet, les caractéristiques anatomiques étant sélectionnées dans le groupe se composant de surfaces de tissus, limites de tissus et texture d'image de structures anatomiques ou pathologiques ordinaires du sujet.

8. L'appareil selon la Revendication 7, où le processeur est configuré de façon à calculer la position de la sonde d'imagerie par rapport aux points de repère, à un emplacement du sujet ou à une caractéristique anatomique du sujet.

9. L'appareil selon la Revendication 1, où le processeur est configuré de façon à fusionner la combinaison de signaux au moyen d'une ou de plusieurs techniques parmi des techniques d'odométrie inertielle visuelle, des techniques d'appariement et de localisation simultanés, des techniques de calage d'images, ou toute combinaison de celles-ci.

10. L'appareil selon la Revendication 9, où le processeur est configuré de façon à :
traiter des données d'images par ultrasons provenant du transducteur à ultrasons, et
réaliser une détermination de la position de la sonde d'imagerie à partir des données d'images par ultrasons traitées.

11. Un procédé d'échographie, le procédé comprenant :
le recueil (310, 410) de données d'image d'un environnement dans lequel un appareil d'échographie est destiné à être actionné, l'appareil d'échographie comprenant un processeur et une sonde d'imagerie qui comprend un ou plusieurs transducteurs à ultrasons, un ou plusieurs capteurs inertiels et un ou plusieurs capteurs optiques, le processeur étant couplé en communication aux un ou plusieurs transducteurs à ultrasons, aux un ou plusieurs capteurs inertiels et aux un ou plusieurs capteurs optiques, l'appareil d'échographie étant configuré de façon à exécuter une échographie médicale non invasive, **caractérisé en ce que** le procédé comprend en outre :
le calibrage (420) de sorties des un ou plusieurs capteurs inertiels par la correction d'une accumulation d'erreurs de dérive des un ou plusieurs capteurs inertiels au moyen d'une combinaison de signaux reçus des un ou plusieurs transducteurs à ultrasons et des un ou plusieurs capteurs optiques, et
l'estimation, avec le processeur, d'une position de la sonde d'imagerie en fonction des sorties calibrées des un ou plusieurs capteurs inertiels.

12. Le procédé selon la Revendication 11, comprenant en outre :
la fusion, avec le processeur, de la combinaison de signaux au moyen d'une ou de plusieurs techniques parmi des techniques d'odométrie inertielle visuelle, des techniques d'appariement et de localisation simultanés, des techniques de calage d'images, ou toute combinaison de celles-ci.

13. Le procédé selon la Revendication 11, où :
les données d'image comprennent des sorties provenant d'un élément ou des deux parmi les capteurs optiques et les transducteurs à ultrasons,
le processeur est configuré de façon à traiter les données d'image de façon à sélectionner une pluralité de points de repère, les points de repère comprenant un élément ou les deux parmi :
un ou plusieurs points, bords ou angles de surfaces ordinaires, accessoires ou objets d'une pièce dans laquelle l'appareil est destiné à être utilisé de façon à examiner un sujet, et
une ou plusieurs caractéristiques anatomiques du sujet, les caractéristiques anatomiques étant sélectionnées dans le groupe se composant de surfaces de tissus, limites de tissus et texture d'image de structures anatomiques ou pathologiques ordinaires du sujet, et
le processeur est configuré de façon à déterminer la position de la sonde d'imagerie par rapport aux points de repère,

14. Le procédé selon la Revendication 11, comprenant en outre l'utilisation (450) de la position déterminée de façon à fournir, à un opérateur de l'appareil, un guidage de navigation destiné à un déplacement de la sonde d'imagerie.

15. Un support lisible par ordinateur non transitoire possédant un logiciel conservé en mémoire sur celui-ci, le logiciel contenant des instructions d'échographie, les instructions amenant l'appareil à exécuter un procédé selon l'une quelconque des Revendications 11 à 14.
